# EUROPEAN PATENT SPECIFICATION

(11) **EP 3 031 518 B1**
(45) Date of publication and mention of the grant of the patent: **20.01.2021**
(21) Application number: 15198257.6
(22) Date of filing: 07.12.2015
(51) Int. Cl.: B01F 5/06, B01F 13/00, B01L 3/00

(54) **FLUID MIXING STRUCTURE, CONTINUOUS REACTION UNIT, CONTINUOUS REACTION REACTOR AND METHOD OF USING THE SAME**
FLÜSSIGKEITSMISCHSTRUKTUR, EINHEIT FÜR KONTINUIERLICHE REAKTION, REAKTOR FÜR KONTINUIERLICHE REAKTION UND VERFAHREN ZUR VERWENDUNG DAVON
STRUCTURE DE MÉLANGE DE FLUIDES, UNITÉ DE RÉACTION CONTINUE, RÉACTEUR DE RÉACTION CONTINUE ET SON PROCÉDÉ D'UTILISATION

(30) Priority: 08.12.2014 EP 14196803
(43) Date of publication of application: 15.06.2016
(73) Proprietor: Lonza Ltd, 3930 Visp (CH)
(72) Inventor: Roberge, Dominique, 3960 Sierre (CH); Plouffe, Patrick, Gatineau Quebec, J8P 1B2 (CA); Macchi, Arturo, Ottawa Ontario, K1L 0A9 (CA)
(74) Representative: Greiner, Elisabeth

(56) References cited:
- CN-A- 102 350 286
- DE-A1- 10 103 425
- US-A1- 2005 220 668
- US-A1- 2008 085 551

## Description

The present invention relates to a fluid mixing structure, a continuous reaction unit, a continuous reaction reactor and a method of using same.

Continuous flow technology can be used, for example, in pharmaceutical research and initial process development instead of traditional batch operations. In some instances, it is advantageous to use a specific type of reactor, i.e. a micro-reactor, in which specific dimensions of process fluid channels formed therein are comparatively small. Advantageously, a reactor of the above described type is scalable, from smaller flow rates (micro-reactor) to higher flow rates, where for scale-up or scale-down, the laws of fluid physics require an appropriate modification of the design of the process fluid channels to achieve equivalent flow regimes (flow patterns) and, therefore, reaction conditions. For example, the smaller dimensions of micro-reactors increase their surface-to-volume ratio which enhances wall-fluid heat transfer rates what in turn allows more severe reaction condition. However, synthesis rates in smaller process channels often result in a laminar flow regime where transport phenomena are diffusion driven and thus relatively slow.

WO 2009/009129 A1 describes an approach by combining parallel rows of mixers (the term used therein for the inventive "mixing structures") through which the increased flow rate is divided. This approach is subject to various problems in the case of multiphase reactions. The reason is that the uniform distribution of flow between the numerous parallel rows of mixers can hardly be ensured without control systems due to pressure fluctuations generated by the moving interface between the continuous and dispersed phases. Consequently, each row of mixers may have different flow rates and reactant ratios. Further, the spade-like fluid mixing structure disclosed in WO 2009/009129 A1 has a symmetric shape which per se has the disadvantage of a stronger tendency for bubbles to re-join and coalesce. In addition, the symmetrical curvatures of this structure are especially adapted to produce a flow regime akin to parallel flow for a specific range of flow rates. The centrifugal forces in such curvatures push the denser fluid towards the outside perimeter, which may favor the segregation of phases into a parallel-like flow regime which is inadequate in multiphase systems.

Within multiphase-systems the flow regime or also called flow pattern is the geometric distribution or topology of the components. The geometry may strongly effect the interfacial area available for mass, momentum or energy exchange between the phases. Moreover, the flow within each phase or component will clearly depend on that geometric distribution.

In order to enhance mixing efficiency, techniques are used to generate secondary flow regimes resulting in a flow field that resembles transitional or turbulent flow rather than laminar flow. This can be done, for example, by means of passive mixing techniques making use of specific geometries of the process fluid channels including curvatures, contraction zones, expansion zones, splits and recombination of streams, and obstacles.

For single phase applications, a contacting element such as a T-junction, for example, may be used to mix the fluidic components, followed by an obstacle-free channel to provide residence time and complete the reaction. In multiphase applications, this approach generally leads to a slug or parallel/annular flow regime after the contacting element. Recirculatory motion within the slug flow homogenizes (i. e. makes more homogeneous or enhances the homogeneity) its interior and reduces the thickness of the boundary layer at the interface, thus increasing mass transfer rate along the channel axis. When parallel flow occurs, the mass transfer depends on the lateral (orthogonal to the channel axis) diffusion of molecules, and high transfer rates are achieved only in smaller geometries where diffusion distances are short and the relative surface area is large.

Alternatively, more complex micro-reactors incorporating multiple consecutive fluid mixing structures may be used. The contacting elements then serve as both the mixer and residence time module. The continuous dissipation of energy can generate and maintain dispersion smaller than slugs throughout the reactor's volume. The drawback of this approach is a generally greater pressure loss and/or lower residence time.

The object of the present invention is to improve the mixing behavior or performance of the fluidic components forming the fluid that flows through continuous reaction reactors such as micro-reactors and, as a consequence, the reaction behavior or behavior of the fluidic components or reactants forming the fluidic components or being part of them.

This object is achieved by the fluid mixing structure of claim 1, the continuous reaction unit of claim 12, the continuous reaction reactor of claim 19, and the method of claim 22. Advantageous modifications are defined in the dependent claims.

It has been found that the fluid mixing structure of the invention and its single passage is particularly useful in case of multiphase reactions. For scale-up, the size of the mixer can be increased according to a 3/7^{th} power rule, and the flow of reactants can be controlled by the feed pumps and needs not to be divided.

The fluid mixing structure of the present invention provides for improved mixing a fluid flow being composed of at least two fluidic components, where the at least two fluidic components, or substances they transport, may react with each other. The fluid flows and the substances are referred herein as process fluid flows and reactants, respectively. That is, the fluid mixing structure of the present invention provides the basis for better reaction conditions between reactants by establishing their intimate contact by letting them pass through the fluid mixing structure. Specifically, the fluid mixing structure of the invention allows mixing of multi-phase fluidic components so as to obtain a mixture wherein one phase is evenly dispersed in another phase.

Generally, the fluidic components to be mixed may be of the same phase or of different phases (single or multi-phase applications).

According to present invention, the fluid mixing structure is continuously passed through by the fluid flow that is generated by mixing at least two feed fluid flows. Specially, the fluid mixing structure may be incorporated in the continuous reaction unit, which in turn may be part of the continuous reaction reactor.

According to the present invention (claim 1), a fluid mixing structure for mixing at least two fluidic components has a flow inlet port and a flow outlet port and comprises a contraction zone, an expansion zone, and a retention zone, arranged in this order in an inflow direction of a fluid flow to flow through said fluid mixing structure and being composed of the at least two fluidic components, and a flow splitter arranged in a space formed by said expansion zone and said retention zone to split said fluid flow in a first sub fluid flow and a second sub fluid flow flowing in a first flow path and a second flow path, respectively, formed in the fluid mixing structure, and to mix said first and second sub fluid flows within said space to generate and discharge a homogenized fluid flow, wherein said flow splitter is arranged and configured to let any flow element of each of said first and second sub fluid flows prior to their mixing have a non-zero average flow component in said inflow direction. The fluid mixing structure is characterized in that in a longitudinal section of the fluid mixing structure, the flow splitter has a polygonal shape or a shape of a flame.

The inventive fluid mixing structure comprises three zones, a first contraction zone, an expansion zone, and a retention zone. The contraction zone is a zone of decreasing flow cross-section (increasing dynamic pressure, decreasing static pressure), the expansion zone is a zone of increasing flow cross-section (decreasing dynamic pressure, increasing static pressure), and the retention zone is a zone of essentially constant cross section. Preferably, each of the walls of the contraction zone extends longitudinally along a straight line for the flow cross-section to decrease linearly in the inflow direction. Preferably, each of the walls of the expansion zone extends along an arc of circle. According to the present invention, the fluid flow flowing through the fluid mixing structure is composed of at least two fluidic components. Prior to flowing into the fluid mixing structure, these two fluidic components are mixed to a certain but insufficient degree. For example, the two fluidic components may originate from different feeding ports, be difficult to mix or may even be immiscible. According to the present invention, the flow splitter serves (i) to split or divide the fluid flow entering the fluid mixing structure in the first and second sub fluid flows, and (ii) to mix - together with walls of the expansion zone and the retention zone - the first and second sub fluid flows to improve their mixing state, i. e. to homogenize the fluid flow. It should be noted that, except for special situations, each of the first and second sub fluid flows contain the at least two fluidic components. According to the present invention, the flow splitter is arranged and configured to let any flow element of each of said first and second sub fluid flows prior to their mixing have a non-zero average flow component in said inflow direction. The flow element is an arbitrarily small, notionally delimited portion or area of the respective flow that is defined by its time-dependent velocity (a vector) and forms the basis for a flow line the flow element follows. That is, the flow splitter is arranged and configured to assure a forward-directed flow of each of the first and second sub fluid flows. In other words, the flow splitter is arranged and configured such that each of the sub fluid flows is deviated with respect to the inflow direction by an angle smaller than 90°. The deviation of an angle of less than 90° is important to avoid formation of strong centrifugal forces which may induce fluid coalescence due to density differences of fluids. Arrangement and configuration parameters of the flow splitter are its general shape (triangle, parallelogram, etc.), size, orientation, and position within the space. Orientation and size, in turn, also depend on the size of the space and flow parameters (flow rate and viscosity of the fluids, density and interfacial tension for multiphase systems).

Advantageously the inventive fluid mixing structure avoids curves and uses the above and below described mixing structures. The inventive mixing structure has experimentally proved to be able to generate droplets having a surface area of up to 300,000 m²/m³ (compared with 14,000 m²/m³ as published by Chevalier et al., Chim. Oggi. 26 (200) 38-24, or 11,000 m²/m³ published by Nieves-Remacha et al., Ind. Eng. Chem. Res. 51 (2012) 16251-16262).

The longitudinal section of the fluid mixing structure is a section essentially parallel to the inflow direction and includes, in the symmetric arrangement of the zones as described above, its symmetry axis. The longitudinal section of the fluid mixing structure is the same section as the longitudinal section of the process fluid channel system introduced in claim 12, of which the fluid mixing structure is part of. That is, center lines of the channels of the process fluid channel system lie in the plane of the longitudinal section, so that the course of the channels can be described by two parameters only (which allows, as a matter of course, a variability of the thickness of the channels).

According to an advantageous modification (claim 2), the at least two flow paths differ in shape and/or length to dephasedly mix the first and second sub fluid flows within said space.

Here, "dephasedly" means that the two sub fluid flows, due to the different flow paths, prior to their mixing cover different distances. The dephasing is important at mixing start when still large drops are present because it avoids coalescence of those large drops downstream of the flow splitter. Preferably, the dephasing is achieved either in that the flow splitter, although having a mirror symmetry and being arranged in such a way that its symmetry axis coincides with the inflow direction, is placed off-axial with respect to that direction, and/or a flow splitter with such a shape is arranged in such a way that those axes do not coincide and/or the flow splitter has an asymmetric shape which is different from the prior art. It should be noted that the inflow direction is defined as the mean flow direction of the fluid flow flowing through the contraction zone. Constructively, in the preferred case where the zones of the fluid mixing structure in a longitudinal section thereof have mirror symmetry, the inflow direction coincides with the line of symmetry. The flow splitter may be compared to a beam splitter in optics generating two beams that are combined after they have covered different optical paths.

According to an advantageous modification (claim 3), the polygonal shape has a first side facing towards said contraction zone and acting as a baffle splitting said fluid flow in said first and second sub fluid flows, a second side extending along said first sub fluid flow, and a third side extending along said second sub fluid flow and being connected with said second side to form a tip pointing in a general down-flow direction.

The flow splitter may be generally compared with an arrowhead pointing in a general downstream direction (i. e. a direction having a component in the inflow direction) that may be defined by a bisecting line of a head angle formed by the second and third sides thereof. It should be noted that, according to the present modification, the first side acting as the baffle may be composed of more than one, plane or curved, surface (cf. below), where two adjacent of these surfaces may or may not be connected by rounded edges. The baffle acts as a stream divider that, as noted above, has to be arranged and configured in such a way that the sub fluid flows are redirected by an angle smaller than 90°. Specifically, the distance between the flow splitter and the first contraction zone has to be made comparatively large in case the first side is made of a single surface extending perpendicularly to the inflow direction, and can be made comparatively small in case of convex shape of the first side. It is evident from the above, that the first side is not allowed to have a concave shape like a spade or shovel that opens in an upstream direction of the process fluid flow.

According to an advantageous modification (claim 4), said second and first sides form sides of a triangle.

According to an advantageous modification (claim 5), the triangle is an isosceles triangle.

Although the triangle according to this advantageous modification is defined to be an isosceles triangle, the triangle may have any shape as long as (i) the first side is adapted to act as a baffle flow-splitting the fluid flow entering the mixing structure, and (ii) the boundary condition that the first and a second sub fluid flow are mixed behind it is fulfilled. In the case of dephasedly mixing the sub fluid flows, the sub-passages corresponding to the first and second sub fluid flows, respectively, have different shapes, sizes, and/or lengths.

It should be noted that in a case of an isosceles triangle, provided the zones and their arrangement are symmetric, the first and second sub fluid flows can be mixed dephasedly only by adjusting the position and/or the orientation of the flow splitter appropriately. Preferably, the triangular flow splitter is shifted perpendicularly away from a symmetric arrangement and/or is rotated out of its symmetric arrangement.

According to an advantageous modification (claim 6), the triangle is a right angle triangle, with one of the second and third sides forming a cathetus and extending parallel to the inflow direction, and the other one of the second and third sides forming the hypothenuse.

The mixing structure according to this advantageous modification may be thought of as being created by starting from an isosceles triangle whose symmetry axis coincides with a straight center line (parallel to the inflow direction) of the mixing structure, and whose point angle is shifted such that its second or third side becomes parallel to the center line to form one of the cathetuses (while the other one of the cathetuses is formed by the first side). It should be mentioned here that in all cases where the first side is made of a single surface (without apex), the triangle is preferably not rotated because a non-perpendicularity between the first side and the inflow direction would possibly violate the above mentioned boundary condition.

According to an advantageous modification (claim 7), the first side has an apex pointing in a direction opposite to the inflow direction, the apex serving as a respective flow splitting point.

In contrast to the above, the first side is a two-surface side in order to improve the flow splitting. As noted above, in this case, the mixing structure can be advanced to the first contraction zone compared to the one-surface side case discussed above. This structure has three additional degrees of freedom, two degrees of freedom of position and one degree of freedom of structure: (i) the angle between the apex forming surfaces, (ii) the distance of the apex with respect to the center line, and (iii) the rotational angle of the flow splitter as a whole with respect to the center line.

According to an advantageous modification (claim 8), the shape is a parallelogram pointing with one of its acute-angled tips in a general upflow-direction of the fluid flow.

The general upflow-direction is a direction having a component in a direction opposite to the inflow direction. The general upflow-direction coincides may be defined to coincide with a diagonal of the parallelogram going through the one of its acute-angled tips.

According to an advantageous modification (claim 9), at least one of the at least two fluidic components is a liquid.

According to advantageous modifications (claim 10), at least one of the at least two fluidic components is a gas. That is, each of the two fluidic components may be either a liquid or a gas, so that the fluid flow may be a gas-gas-flow, a gas-liquid-flow, or a liquid-liquid-flow.

According to advantageous modifications (claim 11), said fluid mixing structure is made at least partly of metal.

Metal has an excellent thermal conductivity (greater than glass, for example) and allows, therefore, a better heat release. This in turn enables to perform reactions with highly concentrated alkaline solutions, for example, that glass cannot withstand.

According to the present invention, the mixing structure my be part of a continuous reaction unit having formed therein a process fluid channel system, comprising the mixing structure, for continuous reaction of a plurality of reactants, or to intensify extraction processes of fluidic components fed into the continuous reaction unit as feed fluid flows to form at least one product flowing out of the continuous reaction unit as a product fluid flow.

That is, according to this modification, the fluid mentioned so far is specified to be a process fluid flowing in the process fluid channel system of the continuous reaction unit, where "reaction" describes a chemical reaction. The continuous reaction unit is a device in which chemical reactions continuously take place within process fluid channels forming an essential part of the process fluid channel system which is adapted to guide a plurality of process fluids, each of which may be either a liquid or a gas. The continuous reaction unit, therefore, stands in contrast to so-called batch-units. The process fluid channels typical have lateral dimensions in the order of a few microns to a few millimeters, depending on the reactions / reactants, the size of the continuous reaction unit, the flow rate, for example. According to the present modification, a plurality of reactants is fed into the continuous reaction unit as feed fluid flows. That is, each of the feed fluid flows may either form itself one of the plurality of reactants, or serves as a carrier medium transporting one or more of the plurality of reactants. Preferably, two or more initial feed fluid flows of the plurality of feed fluid flows may form or contain reactants participating in a first reaction to form a first product, while one or more further ones of the plurality of feed fluid flows may be coupled to a channel after a first, a second, etc. intermediate product has already been formed. That is, in case A1, A2... An are n feed fluid flows that mix to form, by a first chemical reaction taking place, a first intermediate product fluid flow P1, then a further one of the plurality of feed fluid flows may be combined with P1 to form a second intermediate product flow P2 or the above defined product fluid flow P, and so on. Preferably, A1-An initial feed fluid flows combine to form P1-Pm product feed flows, with m < n. It should be noted, in view of the fact that each of the feed fluid flows may either form itself one of the plurality of reactants, or serves as a carrier medium transporting one or more of the plurality of reactants, as noted above, the number of feed fluid flows may differ from the number of reactants.

According to the present invention (claim 12), a continuous reaction unit has formed therein a process fluid channel system comprising at least one reaction passage formed by at least one fluid mixing structure according to one of claims 1 to 11.

Therefore, the reaction unit comprises the process fluid channel system that comprises at least one reaction passage, which in turn comprises at least one fluid mixing structure which in turn comprises the contraction zone, the expansion zone, and the retention zone.

According to an advantageous modification (claim 13), the continuous reaction unit comprises at least one residence passage.

Together, therefore, according to this modification: The reaction unit comprises the process fluid channel system that comprises at least one reaction passage and at least one residence passage, where the at least one reaction passage comprises at least one fluid mixing structure which in turn comprises the contraction zone, the expansion zone, and the retention zone. The residence passage is a passage with no mixing structures (also called "obstacle-free" above) that gives the reactants time to react with each other. Even in the residence passage, however, mixing will occur due to diffusion. In fact, the residence passage will favor mixing because coalescence will not be favored. It should be noted, however, that, depending on the reactivity of the reactants in the given environment, the reactions take already place within the fluid mixing structure. That is, the occurrence of chemical reactions usually cannot be locally separated in correlation with the structure of the continuous reaction unit. Preferably, the at least one reaction passage and at least one residence passage are aligned. Alternatively, especially in view of the above described inventive possibility to arrange the feed fluid flows and product fluid flows, the at least one reaction passage and at least one residence passage may not be linearly arranged but form a channel network. Preferably, at least a part of at least one of the at least one reaction passage is arranged along a straight line. Most preferably, each of the at least one reaction passage is arranged along a straight line, i. e. no mixing structures are arranged in curved passages. Preferably, the depth of the process fluid channels forming the residence passages is deeper than of those forming the reaction passages to gain more volume and enable residence time. The process fluid channels typically have lateral dimensions in the order of a few micrometers to a few millimeters, depending on the reactions / reactants, the size of the continuous reaction unit, the flow rate, etc. Preferably, the depth of the process fluid channels forming the residence passages is about 2 mm, whereas the depth of those forming the reaction passages is about 1,25 mm.

According to an advantageous modification (claim 14), the at least one reaction passage and the at least one retention passage are alternatingly arranged within said process fluid channel system.

That is, behind a plurality of mixing structures M forming a reaction passage, that are arranged like pearls of a chain, where an outlet of any of the plurality of mixing structures is directly connected to an inlet of the following one, there follows a retention passage R: MMM R MM R MMMM R...., where the number of Ms in the chain is arbitrary in principle.

According to an advantageous modification (claim 15), the process fluid channel system is a meander-like structure comprising a plurality of straight passages and curved passages, where according to another advantageous modification (claim 16), the at least one reaction passage is arranged within one of the plurality of straight passages.

The curved passage may have any radius of curvature. Preferably, the curved passage is a U-turn, changing the flowing direction about 180°, in order to accommodate in a snake-like fashion with a large total length within a continuous reaction unit of comparatively small size. Preferably, the process fluid channel system is composed of channels having a simple geometric longitudinal shape and connecting the fluid mixing structures. Preferably, the shapes may be arcs and straight lines.

According to an advantageous modification (claim 17), the continuous reaction unit has the shape of a plane-parallel plate, and a longitudinal section plane of said process fluid channel system is parallel to opposite surfaces of said plane-parallel plate.

That is, the continuous reaction unit is a rectangular parallelepiped in which the two largest surfaces are parallel to each other and define a space there between accommodating the process fluid channel system. To put it differently, the process fluid channel system is embedded in that space. The longitudinal section plane is parallel to the two outer surfaces. Preferably, the longitudinal section plane longitudinally intersects the process fluid channel system.

According to an advantageous modification (claim 18), the plane-parallel plate comprises two sub-plates connected to each other at their respective connecting surfaces both coinciding with said longitudinal section plane.

That is, the process fluid channel system is manufactured by combining the two sub-plates each being provided with a portion of the process fluid channel system. Preferably, due to the plane-parallel plate shape of the reaction unit, any cross-section of the channels is rectangular. Specifically in such a case and alternatively to the above, the process fluid channels are completely embedded in only one of the sub-units, while the other one of them serves as a cover or lid. Preferably, the cover is transparent to allow a visual inspection of the reactions and mixing processes taking place within the reaction unit.

According to the present invention, the continuous reaction unit may be part of a continuous reaction reactor.

Preferably, the continuous reaction reactor comprises a plurality of continuous reaction units each preferably having the shape of a plane-parallel plate. Preferably, all of these plates are arranged and attached to each other to form a stack. Preferably, the pluralities of continuous reaction units forming the continuous reaction reactor are operationally connected to act as a single large continuous reaction unit. That is, the continuous reaction reactor may be thought of as a folded large continuous reaction unit. Alternatively, more than one chemical reaction is performed at a time within the continuous reaction reactor by separately grouping the continuous reaction units forming it.

According to the present invention (claim 19), a continuous reaction reactor comprises at least one continuous reaction unit according to one of claims 12 to 18.

According to an advantageous modification (claim 20), the continuous reaction reactor further comprises at least one heat exchange unit comprising a heat exchange fluid channel system for accommodating and guiding a heat exchange fluid to thermally adjust a temperature of the process fluid channel system.

Preferably, the at least one heat exchange unit is similarly designed as the at least one continuous reaction unit. Preferably, each of them has the shape of a plane-parallel plate. Preferably, the at least one continuous reaction unit and the at least one heat exchange unit are alternatingly arranged to form a braced stack. Preferably, the at least one heat exchange units are separately controllable to allow a separate control of the at least one continuous reaction unit.

According to an advantageous modification (claim 21), the reactor is a micro-reactor.

The small scale of micro-reactor increases their surface-to-volume ratio which in turn enhances wall-fluid heat transfer rates allowing more severe reaction conditions. This advantage can potentially be extended to multiphase reactions by allowing intimate contact between the reacting process flows.

According to the present invention (claim 22), a method for mixing a fluid flow comprising at least two fluidic components uses the fluid mixing structure according to one of claims 1 to 11.

According to the present invention (claim 23), a method for continuously forming at least one product as a liquid product flow using the continuous reaction unit according to claims 12 to 18 from a plurality of reactants each fed into the continuous reaction unit as a fluidic feed flow.

According to the present invention, the continuous reaction unit may be part of the continuous reaction reactor.

Further objects and advantageous of the present invention will be better understood by the following detailed description of preferred embodiments with reference to the accompanying drawings. In the drawings,
Figs. 1A to 1D are various flow regimes obtained using n-butanol as solvent for the alkaline hydrolysis of 4-nitrophenyl acetate with varying flow rates in the SZ, the sickle, and the spade mixing structures;
Figs. 2A - 2C are variations of a flow splitter in a mixing structure according to first to third embodiments of the present inventions;
Figs. 3A - 3C are further variations of a flow splitter in a mixing structure according to fourth to sixth embodiments of the present inventions;
Figs. 4A - 4B are variations of a continuous reaction unit comprising a concatenation of the inventive mixing structures according to a seventh and an eighth embodiment of the present invention;
Fig. 5 is a perspective view of a reactor published in WO 2007/112945 A1 of the same applicant to which the present invention may be applied;
Figs. 6A and 6B each show a comparison between the dependencies of the overall K_{c}a coefficient on the total flow rate for the above discussed known mixing structures (SZ, space, and sickle) as well as the structure according to the present invention using n-butanol (Fig. 6A) and toluene (Fig. 6B) as organic solvent;
Figs. 7A and 7B each show a comparison between the dependencies of the overall K_{c}a coefficient on the total flow rate for various obstacles (including no obstacle) using n-butanol (Fig. 7A) and toluene (Fig. 7B) as organic solvent; and
Fig. 8A shows two different obstacles and a structure without obstacle (obstacle-free structure), and Fig. 8B shows a comparison between the dependencies of the overall K_{c}a coefficient on the total flow rate for two different obstacles and the obstacle-free structure shown in Fig. 8A.

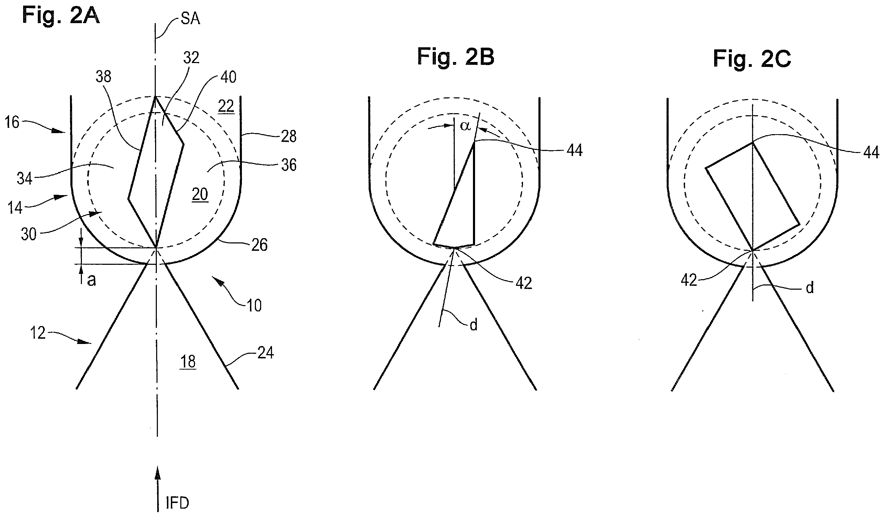

Firstly, a principle design of a fluid mixing structure 10, having the shape of a plane-parallel plate, according to the present invention that is common to all embodiments of Figs. 2A to 3C is representatively described with reference to Fig. 2A (all elements defined by reference numerals in Fig. 2A are also found in the other figures). Figs. 2A to 3C show longitudinal sections of the fluid mixing structure 10.

The fluid mixing structure 10 of Fig. 2A comprises a contraction zone 12, an expansion zone 14, and a retention zone 16 that are mirror-symmetrically arranged along a symmetry axis SA that coincides with an inflow direction IFD of a fluid flow passing through the mixing structure 10 in a down-up-direction of the figure. The height direction of the fluid mixing structure 10 is perpendicular to the plane of protection. The zones 12, 14, and 16 are formed by bottoms 18, 20, and 22, respectively, respective covers (not shown), and side walls 24, 26, and 28, respectively. As shown in Fig. 2A, the longitudinal sections (top views) of the zones 12, 14, and 16, respectively, essentially form a triangle, a semicircle, and a rectangle, respectively. Arranged within a space 30, that is a combination of the expansion zone 14 and the retention zone 16, is arranged a flow splitter 32 splitting or dividing the flow fluid flowing into the fluid mixing structure 10 immediately after having entered the expansion zone 14. The flow splitter 32 separates the space 30 into a left flow path 34 to the left of the flow splitter 32 in Fig. 2A, and a right flow path 36 to the right of the flow splitter 32. The left and right flow paths (for first and second sub fluid flows, respectively) 34 and 36, respectively, mix to a single flow path behind the flow splitter 32. As shown in Fig. 2A, the flow splitter 32 is a distance d away from the interface between the contraction zone 12 and the expansion zone 14. The distance d depends on the specific shape of the flow splitter 32 to be described later, and parameters of the fluid flow (e. g. flow speed) and the fluid itself (e.g. viscosity), and is usually determined and optimized experimentally. It should be noted that dead zones, that usually occur along flow mixing surfaces 38, 40 of the flow splitter 32 and prevent swirling of the fluid flow, are to be avoided as much as possible.

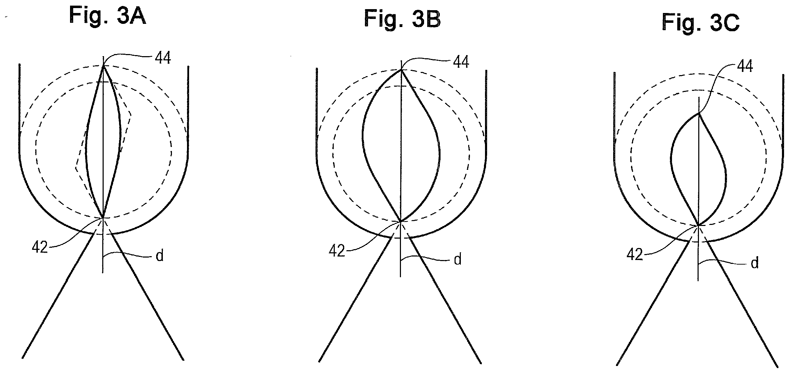

Figs. 2A to 3C show longitudinal sections of the flow splitter 32. That is, the (longitudinal section of the) flow splitter 32 in Fig. 2A is a parallelogram, in Fig. 2B nearly (!) a triangle, in Fig. 2C a rectangle, and in each of Figs. 3A to 3C a flame-like structure.

In all variations, there may be defined a diagonal d (for clarity's sake not shown in Fig. 2A) going through a flow-upstream side apex 42 and a flow-downstream side apex 44 of the flow splitter 32. The diagonal d may be inclined with respect to the symmetry axis SA or the inflow direction IFD by an angle α, as shown in Fig. 2B.

Fig. 2B the flow splitter 32 has the shape of a tetragon as a special case of a polygon, that may be thought of as having been generated from a symmetrically arranged, isosceles triangle whose tip (formed by the two legs of equal length that form its vertex angle) is shifted to the right in Fig. 2B, and whose base (its flow-upstream side acting as a baffle) has formed the kink or apex 42.

In Figs. 3A to 3C the flow splitter 32 has the shape of a flame, that can be thought of as having been generated by a homeomorphism, i. e. by continuously deforming a rectangle that circumscribe the "flames" shown in Figs. 3A to 3C. It is evident from Figs. 3A to 3C that shape and size of the flow splitter 32 can modified arbitrarily to achieve an optimum flow and mixing characteristics.

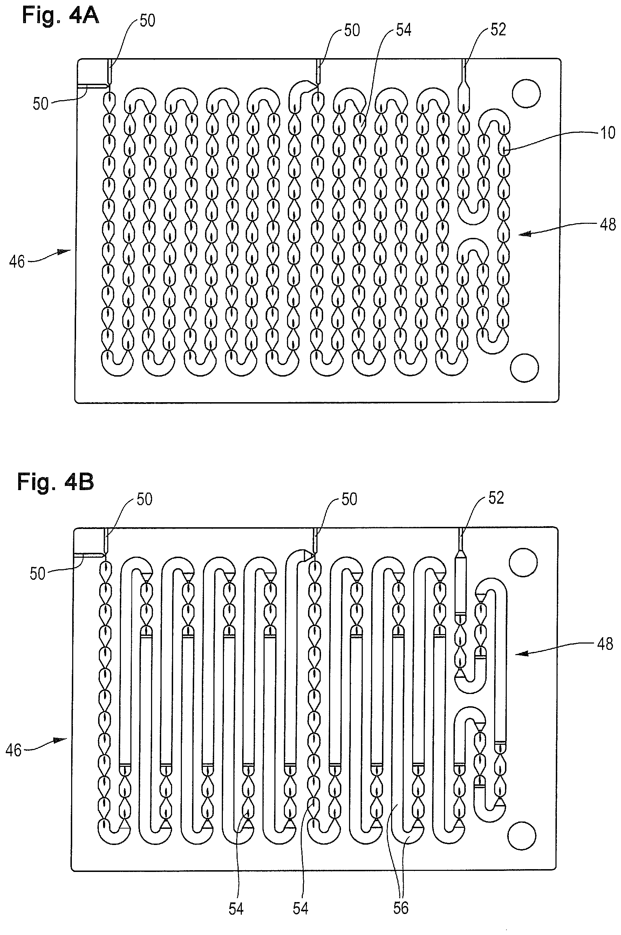

Figs. 4A and 4B are variations of a continuous reaction unit 46 comprising each a concatenation of the inventive mixing structure 10 forming a process fluid channel system 48 comprising feeding ports 50 for introducing feed fluid flows, and an discharging port 52 for discharging a product fluid flow. Specifically, the process fluid channel system 48 comprises a plurality of reaction passages 54 each composed of a plurality of mixing structures 10, and a plurality of residence passages 56 without mixing structures 10. Specifically, the continuous reaction unit 46 shown in Fig. 4A has only U-turn shaped residence passages 56, while all straight passages of the process fluid channel system 48 are formed by a plurality of mixing structures 10. Specifically, no mixing structures 10 are arranged within the U-turns. In contrast, in the process fluid channel system 48 shown in Fig. 4B, the residence passages 56 are also provided within the straight passages. Similarly to the structure shown in Fig. 4A, no reaction passages 54 are arranged within the U-turns.

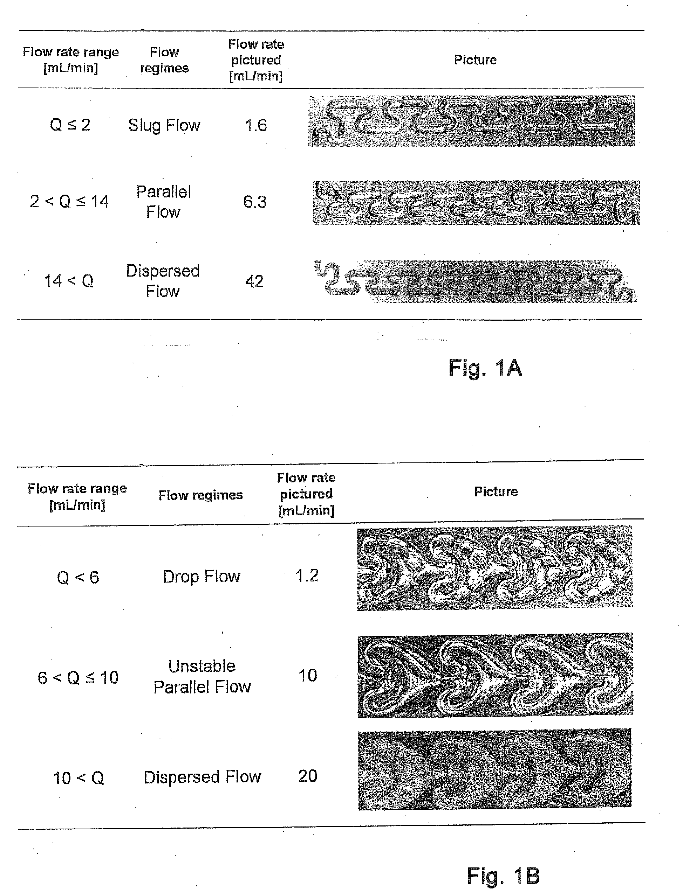

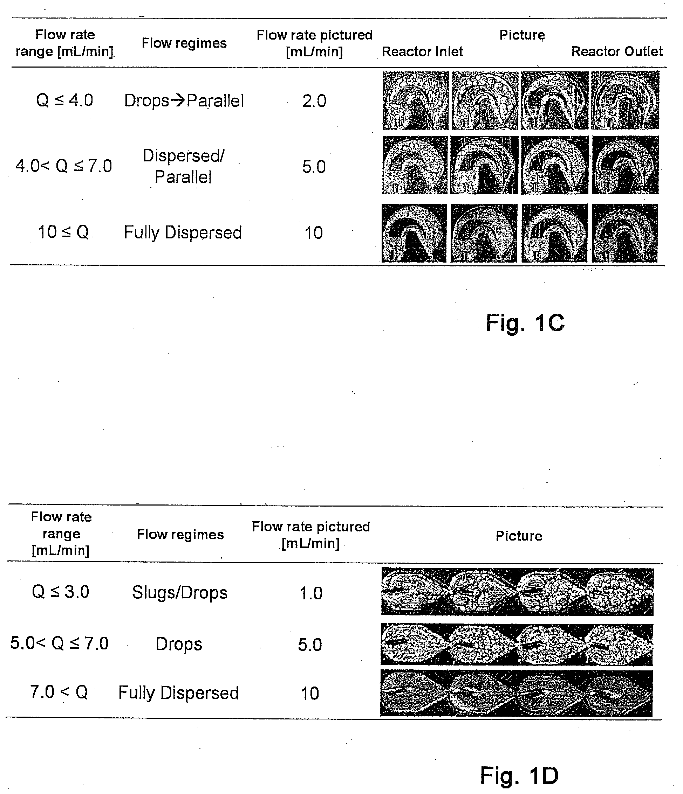

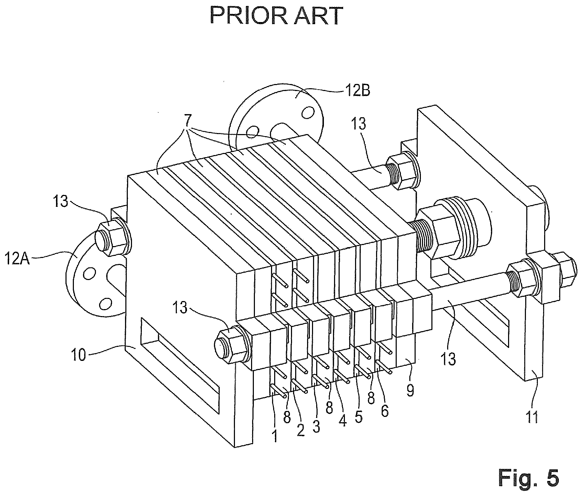

Fig. 5 is a perspective view of a prior art reactor published in WO 2007/112945 A1 of the same applicant (Fig. 1 thereof), to which the present invention may be applied. In this reactor, reference numerals 1 to 6 refer to "process modules" corresponding to the inventive continuous reaction unit 46, and reference numeral 7 refers to "heat exchange modules" that - similarly to an advantageous embodiment of the present invention - are arranged alternatingly with the process modules. That is, according to the present invention, a plurality of the inventive continuous reaction units 46 can be arranged together with a plurality of heat exchange units (in the terminology of the present invention) comprising a heat exchange fluid channel system assuring an optimum temperature regime for the chemical reactions taking place in the continuous reaction units 46.

### Examples

In order to evaluate and compare fluid mixing structures in terms of their mixing performance, test reactions have been carried out. A suitable test reaction for mass transfer investigations must be fast enough to be considered not kinetically limited. The conversion of the reactants is then proportional to the mass transfer rate and allows straightforward calculation of the interphase mass transfer coefficient.

The two-phase alkaline hydrolysis of 4-nitrophenyl acetate: 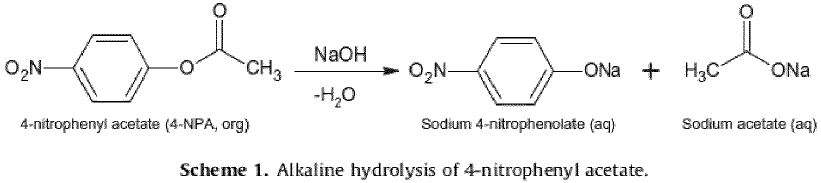 has been used due to its fast intrinsic kinetics and ease of analysis. This fast liquid-liquid reaction allows the direct calculation of the rate of mass transfer and its transport coefficient from the measured conversion. Flow imaging enables identifying the different flow regimes and connecting them to the trends observed in mass transfer.

The acetate is dissolved in an organic solvent and hydrolyzed by an aqueous alkaline solution. The outlet is quenched in a solution of acetic acid, acetonitrile and water and analyzed by HPLC. By using n-butanol and toluene as the organic solvent, a wide range of phase physical properties are covered and provide a baseline for the fluid mixing structures performance in many systems.

Figures 1A, 1B and 1C demonstrate various flow regimes obtained using n-butanol as solvent for the alkaline hydrolysis of 4-nitrophenyl acetate with varying flow rates in structures termed SZ fluid mixing structure (1A), sickle fluid mixing structure (1B), and spade fluid mixing structure (1C). The SZ fluid mixing structure being a serpentine flow channel (Fig. 1A) is well known in the literature and the sickle fluid mixing structure (Fig. 1B) is a mixer combining a triangular obstacle and a curve, both being developed by the present inventors. The spade fluid mixing structure is similar to that disclosed in WO 2009/009129 A1.

It is evident from Figs. 1A, 1B and 1C that all these structures produce a parallel flow regime which is disadvantageous in terms of mixing and, therefore, in terms of reaction. In addition for the spade-like structure it has been observed by Woitalka et al. (Chem. Eng. S., 2014) a stratified flow regime at low flow rate (or long residence time) which correspond to a parallel flow regime.

As shown in Fig. 1D, it is evident that mixing performance according to the present invention (paralleloid or torch mixing structure) does not produce a parallel flow regime, in contrast to the structures discussed above (Figs. 1A to 1C). Additionally, a fully dispersed flow - defined herein as an indistinguishable emulsion indicative of high mass transfer rates - was obtained by the present invention at total flow rates above only 7 mL/min.

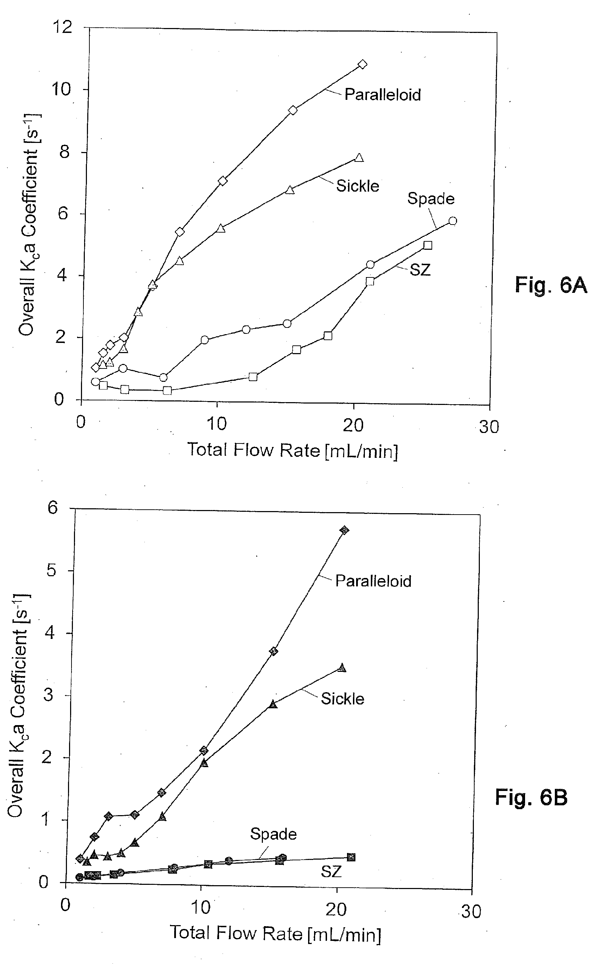

The mixing performance achieved by present invention is more evidently advantageous in Figs. 6A and 6B, which compare the overall continuous phase volumetric mass transfer coefficient K_{c}a for the 4-nitrophenyl acetate hydrolysis as a function of flow rate for the cases of n-butanol (Fig. 6A) and toluene (Fig. 6B) as organic solvent. In Figs. 6A and 6B, a square refers to the SZ mixing structure, a circle refers to the spade mixing structure, a triangle refers to the sickle mixing structure, and a rhombus refers to the polygonal (inventive) mixing structure. It should be noted that the initiation of the parallel flow regime for the SZ and spade structures is clearly observed when a decrease in mass transfer coefficient (K_{c}a) is observed with an increase in the flow rate. Also, the domain of the parallel flow is described in Figs. 1A to 1D.

In both cases, it can be noticed that the mixing performance achieved with the structure of the present invention reaches greater mass transfer coefficients at smaller flow rates. This is due to two particular features unique to the proposed fluid mixing structure. Firstly, it relies on a combination of contraction, expansion, and obstacle to dissipate energy, instead curves, such as in the SZ, spade or sickle mixing structures or the spade fluid mixing structure of WO 2009/009129 A1. Curves submit the fluids to centrifugal forces that promote a radial density gradient and the formation of parallel flow which is inadequate in multiphase systems. Secondly, it relies on an asymmetrical obstacle that brakes the dispersed phase and then desynchronizes its fragments, preventing their coalescence after passing the obstacle and overall ending in smaller drops of the dispersed phase.

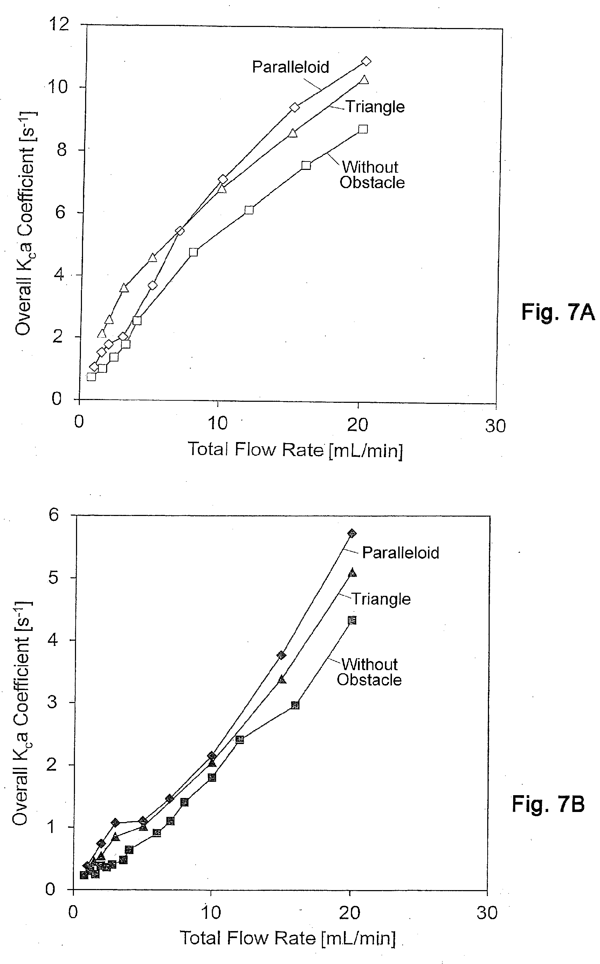

Different variations of the proposed invention are displayed on Figure 7A and further compared on Figs. 7B and 7C. They depict the overall mass transfer coefficient as a function of flow rate for the mixing structure using an obstacle in the shape of a rhombus (also depicted in Figs. 6A and 6B), of a triangle, and without an obstacle. The data for the unobstructed structure unambiguously demonstrate how the specially designed asymmetrical obstacle is necessary to produce favorable flow regimes by breaking the dispersed phase in two and subsequently desynchronizing its fragments. At the lowest flow rates, the structures with obstacle are 2 to 3 times greater. Additionally, the blunt face of the triangular obstacle precipitates the formation of a drop flow regime which increases the mass transfer coefficient at lower flow rates compared to the profiled face of the rhombus when using n-butanol.

Figs. 7A and 7B again compare the overall continuous phase volumetric mass transfer coefficient K_{c}a for the 4-nitrophenyl acetate hydrolysis as a function of flow rate for the cases of n-butanol (Fig. 6A) and toluene (Fig. 6B) as organic solvent, in this case for various obstacles of the inventive structure, i. e. with a rhomboid obstacle (rhombi), with a triangular obstacle (triangles), and without obstacle (squares).

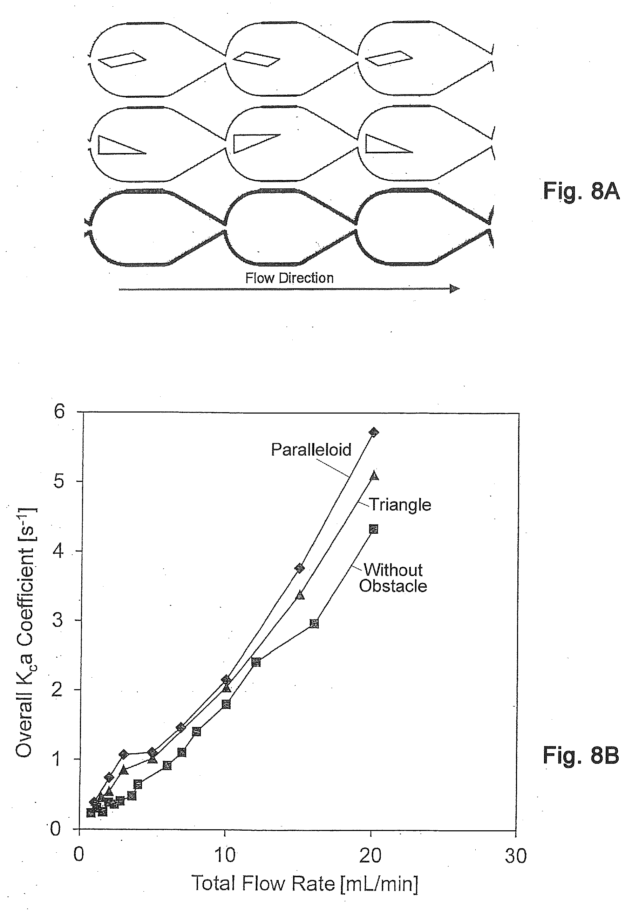

Fig. 8A shows two structures with different obstacles and an obstacle-free structure, and Fig. 8B shows a comparison between the dependencies of the overall K_{c}a coefficient on the total flow rate for two different obstacles and the obstacle-free structure shown in Fig. 8A. It should be noted that although in the middle structure of Fig. 8A, the short cathetus of the triangle facing in a direction opposite to the flow direction is essentially orthogonal to it, due to a stagnation point in front of the obstacle, the "each of said first and second sub fluid flows prior to their mixing have a non-zero average flow component in said inflow direction" as defined in claim 1.

The data for the unobstructed structure (squares) unambiguously demonstrate how the specially designed asymmetrical obstacle is necessary to produce favorable flow regimes by breaking the dispersed phase in two and subsequently desynchronizing its fragments. At the lowest flow rates, the structures with obstacle are 2 to 3 times greater. Additionally, the blunt face of the triangular obstacle precipitates the formation of a drop flow regime which increases the mass transfer coefficient at lower flow rates compared to the profiled face of the rhomboid when using n-butanol.

### List of Reference Numerals

- 10: fluid mixing structure
- 12: contraction zone
- 14: expansion zone
- 16: retention zone
- 18: bottom of 12
- 20: bottom of 14
- 22: bottom of 16
- 24: side wall of 12
- 26: side wall of 14
- 28: side wall of 16
- 30: space
- 32: flow splitter
- 34: left flow path
- 36: right flow path
- 38: flow mixing surface
- 40: flow mixing surface
- 42: flow-upstream side apex of 32
- 44: flow-downstream side apex of 32
- 46: continuous reaction unit
- 48: process fluid channel system
- 50: feeding port
- 52: discharge port
- 54: reaction passage
- 56: residence passage
- SA: symmetry axis
- IFD: inflow direction

## Claims

1. A fluid mixing structure (10) for mixing at least two fluidic components, said fluid mixing structure (10) having a flow inlet port and a flow outlet port and comprising a contraction zone (12), an expansion zone (14), and a retention zone (16), arranged in this order in an inflow direction (IFD) of a fluid flow to flow through said fluid mixing structure (10) and being composed of said at least two fluidic components, and a flow splitter (32) arranged in a space (30) formed by said expansion zone (14) and said retention zone (16) to split said fluid flow in a first sub fluid flow and a second sub fluid flow flowing in a first flow path and a second flow path, respectively, formed in the fluid mixing structure, and to mix said first and second sub fluid flows within said space (30) to generate and discharge a homogenized fluid flow, wherein said flow splitter (32) is arranged and configured to let any flow element of each of said first and second sub fluid flows prior to their mixing have a non-zero average flow component in said inflow direction (IFD)
**characterized in that**:
in a longitudinal section of said fluid mixing structure (10), said flow splitter (32) has a polygonal shape or a shape of a flame.

2. The fluid mixing structure (10) according to claim 1, wherein said at least two flow paths differ in shape and/or length to dephasedly mix the first and second sub fluid flows within said space (30).

3. The fluid mixing structure (10) according to claim 1, wherein said polygonal shape has a first side facing towards said contraction zone (12) and acting as a baffle splitting said fluid flow in said first and second sub fluid flows, a second side extending along said first sub fluid flow, and a third side extending along said second sub fluid flow and being connected with said second side to form a tip pointing in a general down-flow direction.

4. The fluid mixing structure (10) according to claim 3, wherein said second and first sides form sides of a triangle.

5. The fluid mixing structure (10) according to claim 4, wherein said triangle is an isosceles triangle.

6. The fluid mixing structure (10) according to claim 4, wherein said triangle is a right angle triangle, with one of said second and third sides forming a cathetus and extending parallel to said inflow direction (IFD), and the other one of said second and third sides forming the hypothenuse.

7. The fluid mixing structure (10) according to claim 3, wherein said first side has an apex (42) pointing in a direction opposite to said inflow direction (IFD), said apex (42) serving as a flow splitting point.

8. The fluid mixing structure (10) according to claim 1, wherein said shape is a parallelogram pointing with one of its acute-angled tips in a general upflow-direction of the fluid flow.

9. The fluid mixing structure (10) according to one of claims 1 to 8, wherein at least one of said at least two fluidic components is a liquid.

10. The fluid mixing structure (10) according to one of claims 1 to 9, wherein at least one of said at least two fluidic components is a gas.

11. The fluid mixing structure (10) according to one of claims 1 to 10, being made at least partly of metal.

12. A continuous reaction unit (46) having formed therein a process fluid channel system (48) comprising at least one reaction passage (54) formed by at least one fluid mixing structure (10) according to one of claims 1 to 11.

13. The continuous reaction unit (46) according to claim 12, further comprising at least one residence passage (56).

14. The continuous reaction unit (46) according to claim 13, wherein said at least one reaction passage (54) and said at least one retention passage are alternatingly arranged within said process fluid channel system (48).

15. The continuous reaction unit (46) according to one of claims 12 to 14, wherein said process fluid channel system (48) is a meander-like structure comprising a plurality of straight passages and curved passages.

16. The continuous reaction unit (46) according to claim 15, wherein each of said at least one reaction passage (54) is arranged within one of said plurality of straight passages.

17. The continuous reaction unit (46) according to one of claims 12 to 16, wherein said continuous reaction unit (46) has the shape of a plane-parallel plate, and a longitudinal section plane of said process fluid channel system (48) is parallel to opposite surfaces of said plane-parallel plate.

18. The continuous reaction unit (46) according to claim 17, wherein said plane-parallel plate comprises two sub-plates connected to each other at their respective connecting surfaces both coinciding with said longitudinal section plane.

19. A continuous reaction reactor comprising at least one continuous reaction unit (46) according to one of claims 12 to 18.

20. The continuous reaction reactor according to claim 19, further comprising at least one heat exchange unit comprising a heat exchange fluid channel system for accommodating and guiding a heat exchange fluid to thermally adjust a temperature of said process fluid channel system (48).

21. The continuous reaction reactor according to claim 19 or 20, wherein said reactor is a micro-reactor.

22. A method for mixing a fluid flow comprising at least two fluidic components using the fluid mixing structure (10) according to one of claims 1 to 11.

23. The method for continuously forming at least one product as a liquid product flow using said continuous reaction unit (46) according to claims 12 to 18 from a plurality of reactants each fed into said continuous reaction unit (46) as a fluidic feed flow.

## Patentansprüche

1. Fluidmischstruktur (10) zum Mischen von wenigstens zwei fluidischen Komponenten, wobei die Fluidmischstruktur (10) eine Stromeinlassöffnung und eine Stromauslassöffnung aufweist und eine Kontraktionszone (12), eine Expansionszone (14) und eine Retentionszone (16), die in dieser Reihenfolge in einer Zustromrichtung (*inflow direction* - IFD) eines Fluidstroms angeordnet sind, um durch die Fluidmischstruktur (10) zu strömen, und aus den wenigstens zwei fluidischen Komponenten zusammengesetzt sind, und einen Stromteiler (32) umfasst, der in einem Raum (30) angeordnet ist, der durch die Expansionszone (14) und die Retentionszone (16) ausgebildet ist, um den Fluidstrom in einen ersten Teilfluidstrom und einen zweiten Teilfluidstrom zu teilen, die in einem ersten Strömungsweg beziehungsweise einem zweiten Strömungsweg fließen, die in der Fluidmischstruktur ausgebildet sind, und um den ersten und den zweiten Teilfluidstrom innerhalb des Raums (30) zu mischen, um einen homogenisierten Fluidstrom zu erzeugen und abzulassen, wobei der Stromteiler (32) angeordnet und konfiguriert ist, um ein beliebiges Stromelement von jedem des ersten und des zweiten Teilfluidstroms vor deren Mischen eine durchschnittliche Stromkomponente ungleich Null in der Zustromrichtung (IFD) aufweisen zu lassen, **dadurch gekennzeichnet, dass**:
in einem Längsschnitt der Fluidmischstruktur (10) der Stromteiler (32) eine polygonale Form oder eine Form einer Flamme aufweist.

2. Fluidmischstruktur (10) nach Anspruch 1, wobei sich die wenigstens zwei Strömungswege in Form und/oder Länge unterscheiden, um den ersten und den zweiten Teilfluidstrom innerhalb des Raums (30) dephasiert zu mischen.

3. Fluidmischstruktur (10) nach Anspruch 1, wobei die polygonale Form eine erste Seite, die der Kontraktionszone (12) zugewandt ist und als ein Leitblech wirkt, das den Fluidstrom in den ersten und den zweiten Teilfluidstrom teilt, und eine zweite Seite, die sich entlang des ersten Teilfluidstroms erstreckt, und eine dritte Seite aufweist, die sich entlang des zweiten Teilfluidstroms erstreckt und mit der zweiten Seite verbunden ist, um eine Spitze auszubilden, die in eine allgemeine Abwärtsstromrichtung zeigt.

4. Fluidmischstruktur (10) nach Anspruch 3, wobei die zweite und die erste Seite Seiten eines Dreiecks ausbilden.

5. Fluidmischstruktur (10) nach Anspruch 4, wobei das Dreieck ein gleichschenkliges Dreieck ist.

6. Fluidmischstruktur (10) nach Anspruch 4, wobei das Dreieck ein rechtwinkliges Dreieck ist, wobei eine der zweiten und der dritten Seite eine Kathete ausbildet und sich parallel zu der Zustromrichtung (IFD) erstreckt, und die andere der zweiten und der dritten Seite die Hypotenuse ausbildet.

7. Fluidmischstruktur (10) nach Anspruch 3, wobei die erste Seite einen Scheitelpunkt (42) aufweist, der in eine Richtung entgegengesetzt zu der Zustromrichtung (IFD) zeigt, wobei der Scheitelpunkt (42) als ein Stromteilungspunkt dient.

8. Fluidmischstruktur (10) nach Anspruch 1, wobei die Form ein Parallelogramm ist, das mit einer seiner spitzwinkligen Spitzen in eine allgemeine Aufwärtsstromrichtung des Fluidstroms zeigt.

9. Fluidmischstruktur (10) nach einem der Ansprüche 1 bis 8, wobei wenigstens eine der wenigstens zwei fluidischen Komponenten eine Flüssigkeit ist.

10. Fluidmischstruktur (10) nach einem der Ansprüche 1 bis 9, wobei wenigstens eine der wenigstens zwei fluidischen Komponenten ein Gas ist.

11. Fluidmischstruktur (10) nach einem der Ansprüche 1 bis 10, die wenigstens teilweise aus Metall gefertigt ist.

12. Einheit für kontinuierliche Reaktionen (46), in der ein Prozessfluidkanalsystem (48) ausgebildet ist, das wenigstens einen Reaktionsdurchlass (54) umfasst, der durch wenigstens eine Fluidmischstruktur (10) nach einem der Ansprüche 1 bis 11 ausgebildet ist.

13. Einheit für kontinuierliche Reaktionen (46) nach Anspruch 12, die ferner wenigstens einen Verweildurchlass (56) umfasst.

14. Einheit für kontinuierliche Reaktionen (46) nach Anspruch 13, wobei der wenigstens eine Reaktionsdurchlass (54) und der wenigstens ein Retentionsdurchlass innerhalb des Prozessfluidkanalsystems (48) abwechselnd angeordnet sind.

15. Einheit für kontinuierliche Reaktionen (46) nach einem der Ansprüche 12 bis 14, wobei das Prozessfluidkanalsystem (48) eine mäanderartige Struktur ist, die mehrere gerade Durchlässe und gekrümmte Durchlässe umfasst.

16. Einheit für kontinuierliche Reaktionen (46) nach Anspruch 15, wobei jeder des wenigstens einen Reaktionsdurchlasses (54) innerhalb eines der mehreren geraden Durchlässe angeordnet ist.

17. Einheit für kontinuierliche Reaktionen (46) nach einem der Ansprüche 12 bis 16, wobei die Einheit für kontinuierliche Reaktionen (46) die Form einer planparallelen Platte aufweist und eine Längsschnittebene des Prozessfluidkanalsystems (48) parallel zu entgegengesetzten Oberflächen der planparallelen Platte ist.

18. Einheit für kontinuierliche Reaktionen (46) nach Anspruch 17, wobei die planparallele Platte zwei Teilplatten umfasst, die an ihrer jeweiligen Verbindungsoberfläche miteinander verbunden sind, wobei beide mit der Längsschnittebene zusammenfallen.

19. Reaktor für kontinuierliche Reaktionen, der wenigstens eine Einheit für kontinuierliche Reaktionen (46) nach einem der Ansprüche 12 bis 18 umfasst.

20. Reaktor für kontinuierliche Reaktionen nach Anspruch 19, der ferner wenigstens eine Wärmeaustauscheinheit umfasst, die ein Wärmeaustauschfluidkanalsystem zum Aufnehmen und Führen eines Wärmeaustauschfluids umfasst, um eine Temperatur des Prozessfluidkanalsystems (48) thermisch einzustellen.

21. Reaktor für kontinuierliche Reaktionen nach Anspruch 19 oder 20, wobei der Reaktor ein Mikroreaktor ist.

22. Verfahren zum Mischen eines Fluidstroms, der wenigstens zwei fluidische Komponenten umfasst, unter Verwendung der Fluidmischstruktur (10) nach einem der Ansprüche 1 bis 11.

23. Verfahren zum kontinuierlichen Ausbilden wenigstens eines Produkts als einen flüssigen Produktstrom unter Verwendung der Einheit für kontinuierliche Reaktionen (46) nach den Ansprüchen 12 bis 18 aus mehreren Reaktanten, die jeweils der Einheit für kontinuierliche Reaktionen (46) als fluidischer Zufuhrstrom zugeführt werden.

## Revendications

1. Structure de mélange de fluide (10) pour mélanger au moins deux composants fluidiques, ladite structure de mélange de fluide (10) ayant un orifice d'entrée d'écoulement et un orifice de sortie d'écoulement et comprenant une zone de contraction (12), une zone d'expansion (14) et un zone de rétention (16), agencée dans cet ordre dans une direction d'entrée d'écoulement (IFD) d'un écoulement de fluide pour s'écouler à travers ladite structure de mélange de fluide (10) et étant composée desdits au moins deux composants fluidiques, et d'un diviseur d'écoulement (32) agencé dans un espace (30) formé par ladite zone d'expansion (14) et ladite zone de rétention (16) pour diviser ledit écoulement de fluide en un premier écoulement de sous-fluide et un second écoulement de sous-fluide s'écoulant dans un premier trajet d'écoulement et un second trajet d'écoulement, respectivement, formé dans la structure de mélange de fluide, et pour mélanger lesdits premier et second écoulements de sous-fluide à l'intérieur dudit espace (30) pour générer et décharger un écoulement de fluide homogénéisé, dans lequel ledit diviseur d'écoulement (32) est agencé et conçu pour laisser n'importe quel élément d'écoulement de chacun desdits premier et second écoulements de sous-fluide avant leur mélange avoir un composant d'écoulement moyen non nul dans ladite direction d'entrée d'écoulement (IFD)
**caractérisée en ce que** :
dans une section longitudinale de ladite structure de mélange de fluide (10), ledit diviseur d'écoulement (32) a une forme polygonale ou une forme de flamme.

2. Structure de mélange de fluide (10) selon la revendication 1, dans laquelle lesdits au moins deux trajets d'écoulement diffèrent en forme et/ou en longueur pour mélanger de manière déphasée les premier et second écoulements de sous-fluide à l'intérieur dudit espace (30).

3. Structure de mélange de fluide (10) selon la revendication 1, dans laquelle ladite forme polygonale a un premier côté tourné vers ladite zone de contraction (12) et agissant comme un déflecteur séparant ledit écoulement de fluide dans lesdits premier et second sous-écoulements de fluide, un deuxième côté s'étendant le long dudit premier écoulement de sous-fluide, et un troisième côté s'étendant le long dudit second écoulement de sous-fluide et étant relié audit deuxième côté pour former une pointe pointant dans une direction générale d'écoulement descendant.

4. Structure de mélange de fluide (10) selon la revendication 3, dans laquelle lesdits deuxième et premier côtés forment les côtés d'un triangle.

5. Structure de mélange de fluide (10) selon la revendication 4, dans laquelle ledit triangle est un triangle isocèle.

6. Structure de mélange de fluide (10) selon la revendication 4, dans laquelle ledit triangle est un triangle à angle droit, avec l'un desdits deuxième et troisième côtés formant une cathète et s'étendant parallèlement à ladite direction d'entrée d'écoulement (IFD), et l'autre dudit deuxième et troisième côtés formant l'hypoténuse.

7. Structure de mélange de fluide (10) selon la revendication 3, dans laquelle ledit premier côté a un sommet (42) pointant dans une direction opposée à ladite direction d'entrée d'écoulement (IFD), ledit sommet (42) servant de point de division d'écoulement.

8. Structure de mélange de fluide (10) selon la revendication 1, dans laquelle ladite forme est un parallélogramme pointant avec l'une de ses extrémités à angle aigu dans une direction générale d'écoulement ascendant de l'écoulement de fluide.

9. Structure de mélange de fluide (10) selon l'une des revendications 1 à 8, dans laquelle au moins l'un desdits au moins deux composants fluidiques est un liquide.

10. Structure de mélange de fluide (10) selon l'une des revendications 1 à 9, dans laquelle au moins l'un desdits au moins deux composants fluidiques est un gaz.

11. Structure de mélange de fluides (10) selon l'une des revendications 1 à 10, constituée au moins en partie de métal.

12. Unité de réaction continue (46) dans laquelle est formé un système de canaux de fluide de traitement (48) comprenant au moins un passage de réaction (54) formé par au moins une structure de mélange de fluide (10) selon l'une des revendications 1 à 11.

13. Unité de réaction continue (46) selon la revendication 12, comprenant en outre au moins un passage de résidence (56).

14. Unité de réaction continue (46) selon la revendication 13, dans laquelle ledit au moins un passage de réaction (54) et ledit au moins un passage de rétention sont agencés en alternance à l'intérieur dudit système de canaux de fluide de traitement (48).

15. Unité de réaction continue (46) selon l'une des revendications 12 à 14, dans laquelle ledit système de canaux de fluide de traitement (48) est une structure en forme de méandre comprenant une pluralité de passages rectilignes et de passages courbes.

16. Unité de réaction continue (46) selon la revendication 15, dans laquelle chacun desdits au moins un passage de réaction (54) est agencé à l'intérieur de l'un de ladite pluralité de passages rectilignes.

17. Unité de réaction continue (46) selon l'une des revendications 12 à 16, dans laquelle ladite unité de réaction continue (46) a la forme d'une plaque plane parallèle, et un plan de coupe longitudinale dudit système de canaux de fluide de traitement (48) est parallèle aux surfaces opposées de ladite plaque plane parallèle.

18. Unité de réaction continue (46) selon la revendication 17, dans laquelle ladite plaque plane parallèle comprend deux sous-plaques reliées l'une à l'autre au niveau de leurs surfaces de liaison respectives coïncidant toutes deux avec ledit plan de coupe longitudinal.

19. Réacteur de réaction continu comprenant au moins une unité de réaction continue (46) selon l'une des revendications 12 à 18.

20. Réacteur de réaction en continu selon la revendication 19, comprenant en outre au moins une unité d'échange de chaleur comprenant un système de canaux de fluide d'échange de chaleur pour recevoir et guider un fluide d'échange de chaleur pour réguler thermiquement une température dudit système de canaux de fluide de traitement (48).

21. Réacteur de réaction en continu selon la revendication 19 ou 20, dans lequel ledit réacteur est un microréacteur.

22. Procédé pour mélanger un écoulement de fluide comprenant au moins deux composants fluidiques à l'aide de la structure de mélange de fluide (10) selon l'une des revendications 1 à 11.

23. Procédé pour former en continu au moins un produit sous forme d'écoulement de produit liquide à l'aide de ladite unité de réaction continue (46) selon les revendications 12 à 18 à partir d'une pluralité de réactifs introduits chacun dans ladite unité de réaction continue (46) en tant qu'écoulement d'alimentation fluidique.
